# FASCICULE DE BREVET EUROPEEN

(11) **EP 2 167 651 B1**
(45) Date de publication et mention de la délivrance du brevet: **15.08.2012**
(21) Numéro de dépôt: 08806099.1
(22) Date de dépôt: 26.06.2008
(51) Int. Cl.: C12N 7/02

(54) **PROCEDE DE PURIFICATION D'UNE SUSPENSION VIRALE**
VERFAHREN ZUR AUFREINIGUNG EINER VIRUSSUSPENSION
METHOD FOR PURIFYING A VIRAL SUSPENSION

(30) Priorité: 27.06.2007 FR 0704607
(43) Date de publication de la demande: 31.03.2010
(73) Titulaire: Sanofi Pasteur, 69367 Lyon Cedex 07 (FR)
(72) Inventeur: BRASS, Olivier, F-69300 Caluire Et Cuire (FR); CONTZEN, Jean-Marc, F-27740 Poses (FR); FRANCON, Alain, F-69690 Bessenay (FR); TARDY, Michel, F-69009 Lyon (FR)
(86) Numéro de dépôt international: PCT/FR2008/051170
(87) Numéro de publication internationale: WO 2009/007608

(56) Documents cités:
- WO-A-03/045568
- WO-A2-2007/067736
- US-A- 4 158 054
- US-A- 6 146 874
- REIMER C B ET AL: "Purification of large quantities of influenza virus by density gradient centrifugation." JOURNAL OF VIROLOGY DEC 1967, vol. 1, no. 6, décembre 1967 (1967-12), pages 1207-1216, XP002452565 ISSN: 0022-538X
- HEINZ F X ET AL: "PREPARATION OF A HIGHLY PURIFIED VACCINE AGAINST TICK-BORNE ENCEPHALITIS BY CONTINUOUS FLOW ZONAL ULTRA CENTRIFUGATION" JOURNAL OF MEDICAL VIROLOGY, vol. 6, no. 3, 1980, pages 213-222, XP002452566 ISSN: 0146-6615
- KUMAR ANANDA ARONE PREM ET AL: "Process standardization for optimal virus recovery and removal of substrate DNA and bovine serum proteins in Vero cell-derived rabies vaccine." JOURNAL OF BIOSCIENCE AND BIOENGINEERING, vol. 94, no. 5, 2002, pages 375-383, XP002452567 ISSN: 1389-1723
- DORIN M. ET AL: "Principles of Continuous Flow Centrifugation" ARTICLE INTERNET, [Online] XP002452568 Extrait de l'Internet: URL:http://www.beckmancoulter.com/vsearch/ parametric.asp?QueryText=continuous+flow&f ormSubmitted=1&QuerySubmit=true> [extrait le 2007-06-01]
- ANONYME: "KII & PKII ULTRACENTRIFUGE SYSTEMS Solutions for Process Development to Industrial Scale Manufacturing" ARTICLE INTERNET, [Online] XP002452569 Extrait de l'Internet: URL:http://www.awst.com/documents/AWST_Pro ductBroch.pdf> [extrait le 2007-05-01]

## Description

La présente invention est relative au domaine de la purification virale, en particulier à un procédé d'obtention de virus à partir d'une suspension virale liquide.

On connait dans le domaine pharmaceutique, et notamment dans le domaine des vaccins des procédés de purification de suspensions virales.

Ainsi, le brevet FR2201079, décrit notamment un procédé de purification de virus à partir d'une suspension virale que l'on introduit dans un rotor de centrifugeuse de type zonal lorsqu'il est à l'arrêt ; après mise en rotation lente du rotor (entre 2000 et 5000 tours par minute), on introduit au moins 2 coussins de liquide de densité différente à la périphérie du rotor ; puis après que le rotor soit rempli par le liquide à traiter, on l'accélère à une vitesse suffisante pour séparer les virus des impuretés, et enfin, après avoir ramené la vitesse du rotor entre 2000 et 5000 tours par minute, on introduit par la périphérie un liquide de densité égale ou supérieure au contenu du rotor, pour décharger le rotor par son conduit central ; et on recueille ensuite les fractions contenant le virus débarrassé des impuretés.

Lors de la rotation, les coussins de densité différente se transforment par diffusion en gradient non linéaire.

La méthode décrite dans ce brevet qui est une méthode discontinue présente cependant l'inconvénient de ne permettre de traiter que des volumes constants et limités de suspension virale, et donc d'obliger à concentrer la suspension virale à purifier auparavant et/ou à reproduire plusieurs fois les mêmes opérations pour traiter un volume important de liquide.

Le fait d'utiliser une telle méthode complique également les tentatives d'avoir un procédé s'effectuant entièrement dans des conditions stériles.

La présente invention a pour objet un procédé d'obtention de virus à partir d'une suspension virale liquide, comprenant du virus de la grippe et de l'ovalbumine dans du liquide allantoïque d'oeuf, selon lequel la suspension virale est soumise à une ultracentrifugation sur gradient de densité,
caractérisé en ce que,
- l'ultracentrifugation est effectuée en continu,
- le gradient de densité est un gradient discontinu comprenant au moins 2 paliers,
- la force centrifuge à laquelle est soumise la suspension virale liquide à l'entrée de l'ultracentrifugeuse est d'une valeur comprise entre 36 000 et 60 000g,
- la force centrifuge à laquelle est soumise la suspension virale à l'extrémité du gradient de densité est d'une valeur comprise entre 90 000 et 120 000g,
- le volume disponible pour la circulation du liquide et le gradient de densité à l'intérieur de l'ultracentrifugeuse est d'environ 8 litres.

Ainsi, il est possible d'intégrer cette étape de purification dans un procédé de fabrication en ligne, ce qui permet de réduire les risques de contamination, et permet une automatisation intéressante d'un point de vue industriel.

De plus, les virus peuvent de façon optimale, pénétrer dans la couche de gradient de densité, alors que les éléments non-désirés de la suspension virale, traversent directement l'ultracentrifugeuse, et sont éliminés, sans pouvoir pénétrer dans le gradient, ou bien pénètrent dans le gradient, mais migrent peu.

Selon une autre caractéristique particulière du procédé selon l'invention, le gradient de densité est un gradient de saccharose en paliers, obtenu en superposant au moins 2 couches de saccharose de densité différente, et une couche de solution tampon.

On obtient ainsi au moins 2 paliers bien différenciés correspondant à des « sauts » de densité qui permettent une bonne séparation des constituants de la suspension virale.

De nombreux avantages de l'invention apparaitront à la lecture de la description détaillée ci-après, en référence aux figures qui représentent de façon schématique un dispositif permettant sa mise en oeuvre.
La figure 1 représente, de façon schématique, un dispositif de mise en oeuvre de l'invention, à l'arrêt.
La figure 2 représente, de façon schématique, un dispositif de mise en oeuvre de l'invention, en fonctionnement.

Un exemple de dispositif d'ultracentrifugation qui convient particulièrement bien à la mise en oeuvre de l'invention est un dispositif tel que celui décrit dans la demande de brevet US 2005/0215410.

Ainsi que cela apparait sur les figures, un tel dispositif est principalement constitué d'un rotor comprenant une enveloppe externe cylindrique 1 constituant une cuve pourvue d'un noyau central 2. La cuve est pourvue dans sa partie inférieure d'une entrée 3 du liquide à traiter, et dans sa partie supérieure d'une sortie 4. Pour des raisons de simplification, les moyens d'entrainement ainsi que les moyens de fixation des différentes parties du dispositif n'ont pas été représentés.

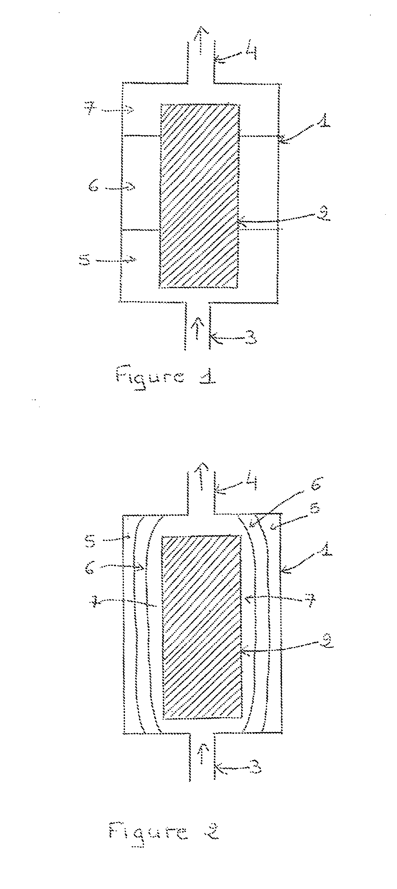

Le volume disponible entre l'enveloppe externe 1 et le noyau 2 est occupé par un gradient de densité, constitué selon l'invention par au moins 2 couches, 5 et 6, de densité différente, et d'une couche 7 de solution tampon. A l'arrêt, les couches du gradient de densité sont superposées à l'horizontale, ainsi que cela est représenté à la figure 1, la couche de plus forte densité se trouvant au-dessous et la couche de tampon se trouvant sur le dessus. Durant la mise en oeuvre du dispositif, ainsi que cela est représenté sur la Figure 2, les couches de densité différente s'organisent de manière concentrique autour du noyau, la couche de plus forte densité étant la plus éloignée du noyau, alors que la suspension virale à traiter qui entre par l'entrée 3 circule dans la zone proche du noyau, avant d'être évacuée par la sortie 4.

Selon l'invention, la suspension virale que l'on traite est une suspension liquide qui contient des virus vivants ou non. Il peut s'agir de virus utilisés dans la fabrication des vaccins, et notamment des virus de la grippe, de la rougeole, des oreillons, de l'herpès, de la fièvre jaune, de la rage, de la polio ou tout autre virus présentant des caractères lui permettant d'être utilisés pour la prévention de maladies. Ces virus peuvent avoir été produits par culture sur des cellules, ou sur des organes, ou comme cela est par exemple le cas avec le virus de la grippe sur des oeufs. Dans le cas de la production de virus sur cellules, les principaux éléments à éliminer seront les protéines et l'ADN cellulaire. Dans le cas de la production sur oeufs, un des éléments qu'il convient d'éliminer est l'ovalbumine. En effet, les normes établies par les autorités de santé en Europe fixent un seuil limite de 1 µg d'ovalbumine par dose de vaccin administré. Bien entendu, les industriels s'attachent à descendre bien en-dessous du seuil autorisé ; il est donc important de pouvoir disposer d'un procédé simple et efficace, permettant de réduire au maximum les impuretés provenant de la production des virus.

Le procédé selon l'invention est d'un intérêt très particulier, et a montré une grande efficacité, dans le cas où la suspension virale à traiter est constituée par du liquide allantoïque comprenant du virus de la grippe qu'il convient de séparer de l'ovalbumine provenant des oeufs utilisés pour la propagation virale.

Le procédé selon l'invention s'effectue en une seule étape d'ultracentrifugation zonale. A cette fin, la cuve d'ultracentrifugation utilisée est une cuve cylindrique dont les parois sont de préférence en titane ; elle est pourvue en son centre, d'un noyau cylindrique muni de rainures permettant l'écoulement de liquide ; ce noyau est avantageusement en Noryl^{™}. Le volume disponible entre le noyau et les parois extérieures est, selon l'invention, suffisant pour introduire un produit permettant un gradient de densité à au moins 2 paliers. Ainsi, on a remarqué qu'en utilisant un système d'ultracentrifugation de type KII avec un noyau K10, on obtenait un volume de 8 litres convenant parfaitement à l'invention.

Afin d'assurer la purification par gradient de densité, la cuve de l'ultracentrifugeuse est chargée à l'arrêt par au moins 3 couches de densité différente. Ce gradient de densité peut être obtenu en utilisant différents produits tels que le saccharose, le chlorure de césium, le tartrate ou le bromure de potassium par exemple ; on a obtenu de particulièrement bons résultats avec le saccharose. On peut donc avoir 3 couches dont 2 sont constituées par du saccharose à des concentrations différentes, et une des couches par une solution tampon. En fonction de la nature de la suspension virale à purifier, il est possible de prévoir plus de couches, ce qui permettra d'avoir plus de paliers de différentes densités.

Le chargement est effectué en introduisant tout d'abord le liquide tampon, puis les coussins par ordre de densité croissante ; ce chargement se fait à l'arrêt.

Lorsque tout a été chargé, le rotor est accéléré à sa vitesse maximale, soit entre 35000 et 40500 rpm. Pendant l'accélération, on introduit la suspension virale à traiter par l'entrée 3. Le liquide à traiter s'écoule alors, ainsi que cela est représenté sur la Figure 2 le long du noyau 2 ; à cet endroit, il est soumis à une force centrifuge comprise entre 36000 et 60000 g, alors que l'accélération à proximité de la paroi externe est comprise entre 90000 et 120 000 g; malgré la relative faible force centrifuge à laquelle est soumise le liquide à traiter à son entrée dans l'ultracentrifugeuse, le virus peut pénétrer à l'intérieur du gradient, et l'on n'observe pas ce qui était craint, une perte trop importante de virus. Certaines des protéines ou des particules non désirées pénètrent aussi à l'intérieur du gradient ; cependant, les paliers de densité différente vont constituer des obstacles infranchissables pour certains des éléments présents au départ dans la suspension virale. Les virus étant des particules lourdes, ils vont eux migrer jusqu'à leur point d'isodensité qui est proche de l'extrémité du gradient. Selon l'invention, on obtient une très bonne séparation virus/ovalbumine. On a ainsi obtenu une élimination de 99% de l'ovalbumine grâce à une seule étape d'ultracentrifugation.

Après que tout le volume de suspension virale ait été traité, on récupère le liquide du gradient de densité par fractions de différentes densité, que l'on traite, afin d'isoler les virus des fractions les plus denses.

On peut ensuite inactiver ou traiter les virus de la manière habituelle.

La présente invention permet de façon surprenante de conserver les paliers du gradient de densité malgré la forte accélération et la durée de cette accélération. Les inventeurs avaient en effet craint qu'un volume aussi important qu'un volume de 8 1 ne conduise à une instabilité du gradient, et que l'on ait en outre une perte virale importante à cause d'une vitesse de rotation à l'entrée du dispositif réduite par rapport à ce qui est habituellement pratiqué dans l'art antérieur.

### Exemple : Purification de virus de la grippe à partir de liquide allantoïque d'oeufs.

On dispose d'un système d'ultracentrifugation de type KII ayant en son centre un noyau K10 fournis par Alfa Wasserman.

On introduit dans le rotor à l'arrêt successivement :
- un peu plus de 8 1 de tampon citrate 125 mM,
- 3,4 1 d'une solution de saccharose à 34%, qui déplace une partie du tampon citrate,
- 1,6 1 d'une solution de saccharose à 55%, qui déplace également une partie du tampon citrate.

Puis on met le rotor en accélération jusqu'à une vitesse de 35 000 rpm, et on introduit le liquide allantoïque à traiter, soit 60 litres, avec un débit de 10 litres/heure. Ce liquide allantoïque a été auparavant clarifié et concentré afin d'éliminer une partie des contaminants ; il contient du virus de la grippe que l'on souhaite isoler pour l'introduire dans un vaccin.

L'ultracentrifugation est effectuée pendant une durée de 6 heures puis on rince pendant 1 heure en remplaçant le liquide allantoique par du tampon citrate 125 mM. Cette phase de rinçage permet non seulement d'éliminer les impuretés à l'entrée du gradient, mais aussi et surtout accorde un temps supplémentaire au virus pour migrer dans sa zone d'isodensité.

Après que tout le liquide à traiter ait été ultracentrifugé, on arrête la rotation, et on récolte le gradient en 32 fractions de 250 ml.

Ces fractions sont ensuite analysées en indice de réfraction, en densité optique, et en dosage d'albumine notamment. On observe que l'on obtient une très bonne résolution de la séparation virus/albumine. On conserve les fractions comprenant entre 47 % et 35 % de saccharose, qui sont les fractions comprenant le maximum de virus, et le minimum d'ovalbumine.

Ces fractions sont ensuite rassemblées, et standardisées par dilution à des valeurs constantes de densité optique indicatrices de la teneur en protéine virale avant d'être fractionnées puis inactivées et filtrées pour obtenir une suspension de particules virales splittées inactivées.

On observe que les virus purifiés selon la méthode de la présente invention sont, avant fragmentation, des virus intègres, et que la concentration en ovalbumine dans les fractions récoltées est suffisamment faible pour une utilisation ultérieure de ces virus dans des doses vaccinales, respectant les spécifications de la Pharmacopée Européenne qui exige une quantité d'ovalbumine inférieure à 1 µg/dose.

## Revendications

1. Procédé d'obtention de virus à partir d'une suspension virale liquide comprenant du virus de la grippe et de l'ovalbumine dans du liquide allantoïque d'oeuf, selon lequel la suspension virale est soumise à une ultracentrifugation sur gradient de densité, **caractérisé en ce que**,
- l'ultracentrifugation est effectuée en continu,
- le gradient de densité est un gradient discontinu comprenant au moins 2 paliers,
- la force centrifuge à laquelle est soumise la suspension virale liquide à l'entrée de l'ultracentrifugeuse est d'une valeur comprise entre 36 000 et 60 000g,
- la force centrifuge à laquelle est soumise la suspension virale à l'extrémité du gradient de densité est d'une valeur comprise entre 90 000 et 120 000g,
- le volume disponible pour la circulation du liquide et le gradient de densité à l'intérieur de l'ultracentrifugeuse est d'environ 8 litres.

2. Procédé selon la revendication 1, **caractérisé en ce que** le gradient de densité est un gradient de saccharose, obtenu en superposant au moins 2 couches de saccharose de densité différente, et une couche de solution tampon.

3. Procédé selon une des revendications précédentes, **caractérisé en ce que** le débit de la suspension virale dans l'ultracentrifugeuse est d'environ 10 litres / heure.

4. Procédé selon une des revendications précédentes, selon lequel :
-- après que toute la suspension virale ait été traitée, on récupère le gradient de densité présent dans l'ultracentrifugeuse par fractions de densité croissante ou décroissante,
-- on conserve les fractions les plus denses comprenant le virus.

5. Utilisation d'un dispositif comprenant au moins:
- un rotor capable d'atteindre une vitesse de rotation d'au moins 35000 tours/ minute, comprenant une cuve cylindrique à paroi en titane, et un noyau interne nervuré en Noryl™,
- une entrée du liquide à traiter,
- une sortie du liquide traité,
- un volume disponible entre ladite cuve cylindrique et ledit noyau interne d'environ 8 litres,
pour la mise en oeuvre du procédé selon une des revendications 1 à 4.

## Claims

1. Method for obtaining viruses from a liquid viral suspension comprising flu virus and ovalbumin in egg allantoic fluid, according to which the viral suspension is subjected to density gradient ultracentrifugation, **characterized in that**,
- the ultracentrifugation is carried out continuously,
- the density gradient is a discontinuous gradient comprising at least 2 steps,
- the centrifugal force to which the liquid viral suspension is subjected at the inlet of the ultracentrifuge has a value of between 36 000 and 60 000 g,
- the centrifugal force to which the viral suspension is subjected at the end of the density gradient has a value of between 90 000 and 120 000 g,
- the volume available for the circulation of the liquid and the density gradient inside the ultracentrifuge is approximately 8 litres.

2. Method according to Claim 1, **characterized in that** the density gradient is a sucrose gradient obtained by superimposing at least 2 layers of sucrose of different density, and one layer of buffer solution.

3. Method according to either of the preceding claims, **characterized in that** the flow rate of the viral suspension in the ultracentrifuge is approximately 10 litres/hour.

4. Method according to one of the preceding claims, according to which:
- after all the viral suspension has been treated, the density gradient present in the ultracentrifuge is recovered by fractions of increasing or decreasing gradient,
- the most dense fractions comprising the virus are kept.

5. Use of a device comprising at least:
- a rotor capable of reaching a rotational speed of at least 35 000 rpm, comprising a titanium-walled cylindrical tank and a ribbed inner core made of Noryl™,
- an inlet for the liquid to be treated,
- an outlet for the treated liquid,
- a volume available between said cylindrical tank and said inner core of approximately 8 litres.
for the implementation of the method according to one of Claims 1 to 4.

## Patentansprüche

1. Verfahren zur Herstellung von Virus, ausgehend von einer flüssigen viralen Suspension, die den Grippevirus und Ei-Albumin in der allantoinhaltigen Flüssigkeit von Eiern umfasst, bei dem die virale Suspension einer Ultrazentrifugierung über dem Dichtegradienten unterzogen wird, **dadurch gekennzeichnet, dass**
- die Ultrazentrifugierung kontinuierlich durchgeführt wird,
- der Dichtegradient ein diskontinuierlicher Gradient ist, der mindestens zwei Absätze umfasst,
- die Zentrifugalkraft, bei der die flüssige virale Suspension am Eintritt der Ultrazentrifuge unterzogen wird, einen Wert zwischen einschließlich 36000 und 60000 g besitzt,
- die Zentrifugalkraft, bei der die virale Suspension am Ende des Dichtegradienten unterzogen wird, einen Wert zwischen einschließlich 90000 und 120000 g besitzt,
- das verfügbare Volumen für den Kreislauf der Flüssigkeit und den Dichtegradienten im Inneren der Ultrazentrifuge etwa 8 Liter beträgt.

2. Verfahren nach Anspruch 1, **dadurch gekennzeichnet, dass** der Dichtegradient ein Gradient von Saccharose ist, erhalten durch Überlagerung von mindestens zwei Schichten von Saccharose mit unterschiedlicher Dichte und einer Schicht Pufferlösung.

3. Verfahren nach einem der vorstehenden Ansprüche, **dadurch gekennzeichnet, dass** der Durchsatz der viralen Suspension in der Ultrazentrifuge etwa 10 Liter/Stunde beträgt.

4. Verfahren nach einem der vorstehenden Ansprüche, gemäß dem man
- nachdem die gesamte virale Suspension behandelt wurde, den in der Ultrazentrifuge vorhandenen Dichtegradienten durch ansteigende oder abnehmende Dichtefraktionen gewinnt,
- die dichtesten Fraktionen, die den Virus umfassen, aufbewahrt.

5. Verwendung einer Vorrichtung, umfassend mindestens:
- einen Rotor, der geeignet ist, eine Umdrehungsgeschwindigkeit von mindestens 35000 Umdrehungen/Minute zu erreichen, umfassend einen zylindrischen Behälter mit einer Wandung aus Titan und einen inneren gerippten Kern aus Noryl^{™},
- einen Eintritt für die zu behandelnde Flüssigkeit,
- einen Austritt für die behandelte Flüssigkeit,
- ein verfügbares Volumen zwischen dem genannten zylindrischen Behälter und dem genannten inneren Kern von etwa 8 Liter,
für die Durchführung des Verfahrens gemäß einem der Ansprüche 1 bis 4.
